# FASCICULE DE BREVET EUROPEEN

(11) **EP 0 730 432 B1**
(45) Date de publication et mention de la délivrance du brevet: **12.07.2000**
(21) Numéro de dépôt: 95900820.2
(22) Date de dépôt: 09.11.1994
(51) Int. Cl.: A61B 6/00, A61B 6/04

(54) **APPAREIL D'IMAGERIE MEDICALE POUR EXAMEN RADIOLOGIQUE GENERAL ET/OU CARDIO-VASCULAIRE A VISEE DIAGNOSTIQUE OU THERAPEUTIQUE**
MEDIZINISCHE ABBILDUNGSVORRICHTUNG FÜR ALLGEMEINE UND/ODER KARDIOVASKULÄRE RÖNTGENUNTERSUCHUNGEN MIT DIAGNOSTISCHEN ODER THERAPEUTISCHEN EINSTELLUNGEN
MEDICAL IMAGING APPARATUS FOR PERFORMING A GENERAL AND/OR CARDIOVASCULAR RADIOLOGICAL EXAMINATION FOR DIAGNOSTIC OR THERAPEUTICAL PURPOSES

(30) Priorité: 10.11.1993 FR 9313465
(43) Date de publication de la demande: 11.09.1996
(73) Titulaire: Gaudel, Jacques, 13008 Marseille (FR); Merlin, Jacques, F-13013 Marseille (FR)
(72) Inventeur: Gaudel, Jacques, 13008 Marseille (FR); Merlin, Jacques, F-13013 Marseille (FR)
(74) Mandataire: Roman, Michel
(86) Numéro de dépôt international: FR9401312
(87) Numéro de publication internationale: WO9513017

(56) Documents cités:
- EP-A- 0 049 562
- EP-A- 0 122 849
- EP-A- 0 165 157
- DE-A- 2 046 207
- US-A- 5 014 292

## Description

La présente invention concerne un appareil d'imagerie médicale pour examen radiologique général ou cardio-vasculaire à visée diagnostique ou thérapeutique.

Le domaine technique de l'invention est celui de la construction d'appareils de radiologie.

Compte tenu de l'évolution rapide qui se poursuit en imagerie médicale, il est de plus en plus nécessaire d'offrir au service hospitalier ainsi qu'aux cliniques et cabinets privés un équipement aussi polyvalent que possible. Il est en effet probable que la nécessité d'optimiser le rapport coût/rendement ne permettra plus qu'à quelques grands centres de conserver la variété d'équipement que l'on y trouve actuellement. Ce concept d'installation polyvalente ne peut être réalisé qu'avec le concours des techniques d'acquisition numérique des images radiologiques et à condition de disposer d'un statif télécommandé qui permette une exploration du patient aussi bien en position horizontale que verticale, une exploration longitudinale du patient sur une longueur suffisante, un plateau porte-patient à fixation unilatérale permettant une accessibilité totale à l'une de ses extrémités, un déplacement longitudinal et latéral du patient, un réglage en hauteur du patient par rapport à l'isocentre, une angulation dans le plan transversal de ± 90° au moins et de ± 45° dans le plan cranio-caudal.

Le brevet N° EP-A-0 165 157 (THOMSON CGR) décrit un appareil de radiographie polyvalent comprenant un plateau porte-patient mobile solidaire d'un statif muni d'un bras portant l'équipement radiographique. Il permet entre autre d'effectuer des radiographies dans un plan cranio-caudal sagittal en utilisant la rotation d'un support intermédiaire autour d'un axe horizontal ainsi que des incidences transversales grâce au déplacement circulaire d'un arceau dans son plan.

Cependant cet appareil ne permet pas d'effectuer des prises de vues présentant une angulation de plus ou moins 45° dans un plan cranio-caudal frontal.

Le but de la présente invention est donc de proposer un appareil d'imagerie répondant aux exigences précitées, l'ensemble des mouvements dudit appareil étant susceptible de s'inscrire dans un volume raisonnable compatible avec les dimensions des salles de diagnostic actuelles.

L'invention concerne à cet effet un appareil d'imagerie médicale pour examen radiologique général ou cardio-vasculaire à visée diagnostique ou thérapeutique selon la revendication 1.

Selon des modes préférentiels de réalisation de l'invention :
- le deuxième bras (ou branche) est de longueur variable pour permettre une exploration longitudinale du patient,
- l'équipement radiographique est monté à coulissement le long du deuxième bras pour permettre une exploration longitudinale du patient,
- les bras sont constitués par une seule et pièce comprenant au moins deux branches, l'une montée à pivotement autour du premier axe xx' sur le berceau support, l'autre de longueur éventuellement variable supportant l'équipement radiologique,
- le berceau support de plateau porte-patient est équipé de moyens de coulissement tels que des rails de guidage latéraux courbes (incurvés par exemple en arc de cercle) qui coopèrent avec des moyens de guidage de l'embase tels que des glissières ou galets disposés entre les branches de l'embase permettant au plateau porte-patient d'occuper n'importe quelle position intermédiaire entre la position verticale et la position horizontale sans modifier les autres réglages,
- l'équipement de diagnostic comprend un support incurvé, par exemple en forme de C, monté solidaire du bras par la partie centrale du C et pouvant pivoter selon le deuxième axe yy', les extrémités du C étant respectivement munies du générateur de rayons X et du récepteur d'images qui sont disposés de telle sorte que le foyer de la source de rayons X et le centre du récepteur sont situés sur un troisième axe zz' qui rencontre constamment le premier axe xx' à son point d'intersection avec le deuxième axe yy'.

En d'autres termes, l'invention procure un appareil d'imagerie médicale pour examen radiologique comportant un plateau porte-patient monté solidaire d'une structure articulée motorisée de préférence télécommandée, laquelle structure articulée supporte un équipement de radiographie constitué notamment d'un générateur de rayons X et d'un récepteur d'images X, dans lequel ladite structure articulée comporte un berceau formant support de plateau porte-patient, de préférence en forme de C, c'est-à-dire en forme d'un secteur angulaire d'un tronçon de tube d'axe OO', lequel berceau est monté mobile en rotation selon un axe de rotation horizontal OO' sur une embase ; ledit berceau porte une colonne télescopique suppportant ledit plateau porte-patient qui est monté mobile en translation par rapport audit berceau selon un axe perpendiculaire audit axe de rotation OO' ; ledit berceau porte à proximité de son extrémité supérieure une première extrémité (ou branche) d'un bras coudé qui est montée pivotante autour d'un axe xx' contenu dans un plan vertical et perpendiculaire audit axe de rotation OO', et qui est parallèle à l'axe longitudinal du plateau porte-patient, permettant un débattement angulaire entre ledit bras coudé et ledit berceau de ± (plus ou moins) 90° ; la deuxième extrémité (ou branche) dudit bras coudé reçoit ledit équipement de radiographie qui est monté rotatif par rapport à ladite deuxième extrémité dudit bras coudé selon un deuxième axe yy' de rotation s'étendant dans un plan contenant le "squelette" dudit bras coudé, c'est-à-dire passant par ladite deuxième extrémité dudit bras coudé et étant perpendiculaire audit axe xx' de pivotement de ladite première extrémité par rapport audit berceau, de manière à permettre un débattement angulaire d'au moins ± (plus ou moins) 45° dudit équipement radiographique dans un plan parallèle au plan du plateau porte-patient.

D'autres caractéristiques et avantages de l'invention apparaîtront encore à la lecture de la description qui suit et des dessins joints, dans lesquels:
La figure 1 représente une vue de face de l'appareil conforme à l'invention, et

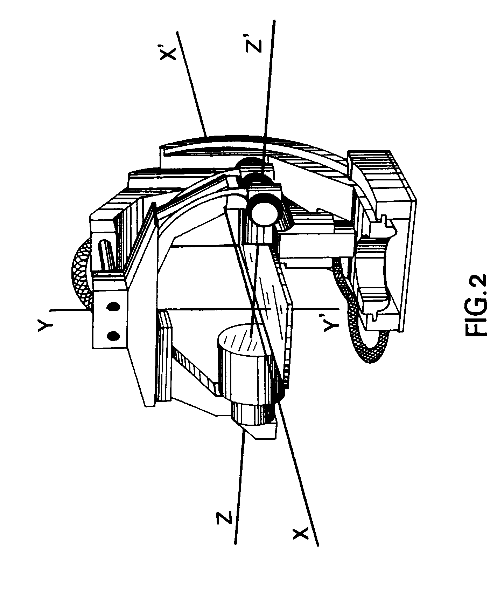

la figure 2 représente une vue de profil de l'appareil conforme à l'invention.

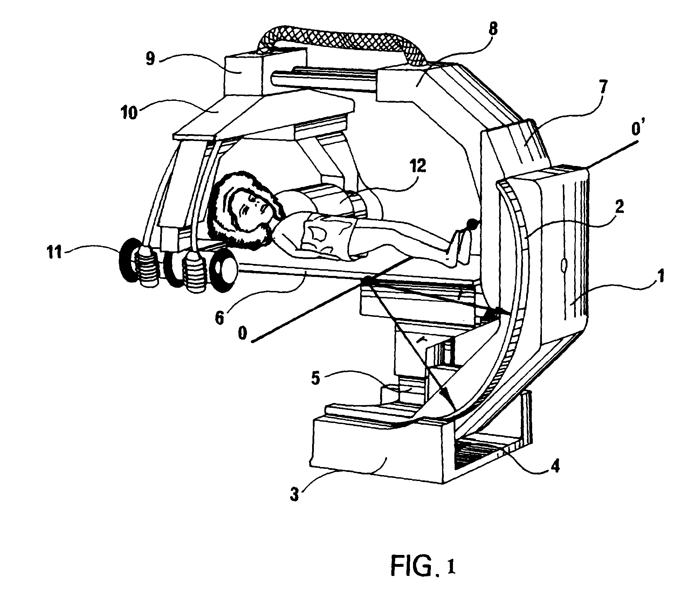

Conformément à la figure 1, l'appareil d'imagerie pour examen radiologique à visée diagnostique ou thérapeutique comprend un plateau porte-patient 6, un support 1 de plateau porte-patient 6 en forme générale de C, plus ou moins ouvert, monté mobile sur une embase 3. Tel que représenté à la figure 1, ce support de plateau porte-patient est monté à coulissement sur l'embase 3 au moyen de rails latéraux 2 en arc de cercle de rayon r qui coulissent dans l'embase 3 en forme générale de U, laquelle comporte sur les faces intérieures de ses branches des glissières 4 ou tout autre moyen de guidage approprié qui permettent au support du plateau porte-patient 1 de pivoter autour d'un axe OO' reliant les centres fictifs des arcs de cercles que constituent les rails latéraux 2. Grâce à ce positionnement du support 1 sur l'embase 3, le plateau porte-patient 6 peut occuper n'importe quelle position intermédiaire entre une position horizontale et une position verticale. Bien évidemment, les moyens de pivotement représentés, à savoir les rails latéraux 2, ne constituent que des exemples de réalisation de ce mouvement de pivotement du support du plateau porte-patient 1 par rapport à l'embase 3.

A l'une des extrémités de ce support de plateau porte-patient 1, est disposée une colonne 5 supportant le plateau porte-patient 6. Entre le plateau porte-patient et la colonne 5 est disposé un mécanisme en soi connu permettant au plateau porte-patient 6, transparent aux rayons X, de se mouvoir selon trois axes. Grâce à ce mécanisme, on obtient une possibilité de déplacement longitudinal et latéral du patient ainsi qu'un réglage en hauteur du patient par rapport à l'isocentre. L'autre extrémité du support 1 de plateau porte-patient est montée solidaire d'un bras 7. Ce bras 7, représenté à la figure 1 en forme générale de C, mais qui pourrait affecter toute autre forme telle qu'une forme d'équerre, est monté à pivotement autour d'un axe xx' parallèle à l'axe longitudinal du plateau porte-patient 6 et parallèle au plan contenant le squelette du support 1. Cette rotation du bras 7 par rapport au support 1 du plateau porte-patient permet une angulation de l'équipement radiographique dans le plan transversal du patient d'au moins ± 90°.

Ce bras 7 est monté lui-même solidaire d'un bras 8 au moyen d'une liaison de préférence rigide. A l'extrémité 9 opposée à celle en liaison avec le bras 7, le bras 8 supporte l'équipement 10 de radiographie. Cet équipement de radiographie est constitué notamment d'un générateur 11 de rayons X, d'un récepteur 12 d'images X. Ce récepteur d'images peut être un écran radioscopique ou une cassette porte-film ou un amplificateur de brillance associé à une chaîne de télévision ou tout autre dispositif connu. Ce générateur de rayons X et ce récepteur d'images X sont maintenus en position au moyen d'un support 10 en forme générale de C monté solidaire du bras 8 par la partie centrale du C, les extrémités du C étant respectivement munies du générateur 11 de rayons X et du récepteur 12 d'images X qui sont disposés de telle sorte que le foyer de la source de rayons X et le centre du récepteur sont situés sur un axe zz' qui rencontre constamment l'axe xx' à son point d'intersection avec un axe yy'. Cet axe yy' correspond en fait à l'axe de rotation du support 10 de l'équipement radiographique 10, 11, 12 par rapport au bras 8. Cet axe yy' rencontre constamment à angle droit l'axe xx' quelle que soit la position angulaire du bras 7 et la position du bras 8. Grâce à cette rotation du support 10 portant l'équipement radiologique autour de l'axe yy', on obtient une angulation d'au moins ± 45° dans le plan cranio-caudal. On pourrait également imaginer, dans une autre forme de réalisation de l'invention, que les bras 7 et 8 soient constitués par une seule et même pièce comprenant au moins deux branches, l'une montée à pivotement autour de l'axe xx' sur le support 1, l'autre de longueur éventuellement variable supportant l'équipement radiologique 10, 11, 12. Dans ce cas, l'équipement radiologique 10, 11, 12 pourrait conserver la même forme. Pour permettre également une exploration longitudinale du patient sur une longueur suffisante, généralement au moins 1,40m, on prévoit que le bras 8 soit de longueur variable, c'est-à-dire tel que représenté à la figure 1, ce bras 8 étant équipé de tiges téléphoniques qui relient les deux extrémités du bras 8 permettant ainsi une variation de longueur du bras 8. Il est également possible d'envisager que l'équipement radiographique 10, 11, 12 et plus particulièrement son support 10 soient montés à coulissement le long du bras 8. On notera que, dans le mode de réalisation représenté à la figure 1, le plateau porte-patient 6 est à fixation unilatérale puisqu'il est solidaire de l'ensemble uniquement au moyen de la colonne 5, ce qui permet une accessibilité totale à l'une de ses extrémités. On notera également qu'en raison de la conception même de ce statif, la répartition des charges autour de l'axe fictif de rotation du support 1 du plateau porte-patient est suffisamment équilibrée pour que le basculement du support ne nécessite qu'un moteur de faible puissance.

Ainsi, pour une exploration totale d'un malade, il suffit, après avoir placé le patient sur le plateau porte-patient 6, c'est-à-dire dans l'axe xx', d'amener l'équipement radiographique 10, 11, 12 en face de la région anatomique à examiner par coulissement de 10 par rapport au bras 8, ou variation de longueur du bras 8, puis de faire pivoter les bras 7 et 10 selon les angles prédéterminés pour être en mesure d'effectuer les examens désirés. Dans le cas où cette exploration nécessiterait un positionnement vertical ou intermédiaire entre la position horizontale et la position verticale du patient, il suffira de commander le basculement du plateau 1 du porte-patient entre les branches de son embase 3. En conséquence, le statif objet de l'invention est d'un maniement rapide et aisé et permet tout type d'exploration.

## Revendications

1. Appareil d'imagerie médicale pour examen radiologique général ou cardio-vasculaire à visée diagnostique ou thérapeutique comportant un plateau porte-patient (6) monté solidaire d'un statif de préférence télécommandé, lui-même supportant un équipement de radiographie constitué notamment d'un générateur de rayons X (11), d'un récepteur d'images X (12) tel qu'un écran radioscopique ou une cassette porte-film ou un amplificateur de brillance associé à une chaîne télévision et de moyens de support du générateur et du récepteur,
caractérisé en ce que le statif comporte un berceau support (1) de plateau porte-patient (6), de préférence en forme générale de C, monté mobile sur une embase (3) et muni à une extrémité d'une colonne (5) supportant ledit plateau porte-patient et à son autre extrémité d'un premier bras (7) monté à pivotement autour d'un premier axe (xx') parallèle à l'axe longitudinal du plateau porte-patient (6), ainsi qu'au plan contenant le squelette du berceau support (1), pour permettre une angulation de plus ou moins 90° dans le sens transversal par rapport au plateau porte-patient (6), ledit premier bras (7) étant lui-même monté solidaire d'un deuxième bras (8) sur lequel est monté à rotation, autour d'un deuxième axe (yy') perpendiculaire au premier axe (xx'), l'équipement radiographique (10, 11, 12) de manière à permettre un déplacement de ce dernier d'une angulation d'au moins plus ou moins 45° dans le plan cranio-caudal, le générateur de rayons X (11) et le récepteur d'images X (12) étant maintenus de part et d'autre du plateau porte-patient au moyen d'un support (10) courbe aux extrémités duquel ils sont respectivement montés, ledit support étant solidaire par sa partie centrale du deuxième bras (8) et pouvant pivoter selon le deuxième axe (yy'), ledit générateur et ledit récepteur étant disposés de telle sorte que le foyer de la source de rayons X et le centre du récepteur sont situés sur un troisième axe (zz') qui rencontre constamment le premier axe (xx') à son point d'intersection avec le deuxième axe (yy').

2. Appareil d'imagerie selon la revendication 1, caractérisé en ce que le plateau porte-patient (6) est monté solidaire d'une structure articulée motorisée comportant un berceau support (1) monté mobile en rotation selon un axe de rotation horizontal (OO') sur une embase (3), ledit berceau support portant une colonne télescopique (5) supportant le plateau porte-patient (6) qui est monté mobile en translation par rapport audit berceau support, lequel berceau support porte un élément coudé constituant le premier bras (7) pivotant autour du premier axe (xx') et le second bras (8) recevant l'équipement radiographique (10, 11, 12) monté rotatif par rapport audit deuxième bras (8) selon un deuxième axe (yy') s'étendant dans un plan contenant le squelette dudit élément coudé.

3. Appareil d'imagerie selon l'une quelconque des revendications précédentes, caractérisé en ce que le deuxième bras (8) est de longueur variable.

4. Appareil d'imagerie selon l'une quelconque des revendications 1 et 2, caractérisé en ce que l'équipement radiographique (10, 11 12) est monté à coulissement le long du deuxième bras (8).

5. Appareil d'imagerie selon l'une quelconque des revendications 1 et 2, caractérisé en ce que le premier bras (7) et le deuxième bras (8) sont constitués par une seule et même pièce comprenant au moins deux branches, l'une montée à pivotement autour du premier axe (xx') sur le support (1), l'autre de longueur éventuellement variable supportant l'équipement radiologique (10, 11, 12).

6. Appareil d'imagerie selon l'une quelconque des revendications précédentes, caractérisé en ce que le berceau support (1) est équipé de moyens de coulissement (2) tels que des rails de guidage latéraux en arc de cercle qui coopèrent avec des moyens de guidage (4) de l'embase (3) tels que des glissières ou galets disposés entre les branches de l'embase (3) permettant au plateau porte-patient (6) d'occuper n'importe quelle position intermédiaire entre la position verticale et la position horizontale.

## Claims

1. Medical imagery equipment for general or cardiovascular medical examination for diagnostic or therapeutic purposes comprising a patient platform (6) attached to a preferably remote-controlled stand, itself supporting radiographic equipment and notably an X-ray generator (11), an X image receiver (12) such as a radioscopic screen or a film cassette or a brightness amplifier associated with a television channel and means for supporting the generator or the receiver,
characterised in that the stand comprises a support cradle (1) for the patient platform (6), preferably C-shaped, fitted so as to be mobile on a base (3) and fitted at one end with a column (5) supporting the said patient platform and at its other end with a first arm (7) fitted so that it pivots around a first axis (xx') parallel to the patient platform's (6) longitudinal axis, and to the plane containing the frame of the support cradle (1), to enable angling by more or less 90° in the transverse direction relative to the patient platform (6), the said first arm (7) being itself fitted with a second arm (8) on which is fitted the radiological equipment (10, 11, 12) so that it rotates around a second axis (yy') perpendicular to the first axis (xx') so as to allow movement of the latter by an angle of more or less 45° in the craniocaudal plane, the X-ray generator (11) and the X images receiver (12) being maintained on either side of the patient platform using a curve support (10) at the ends of which they are respectively fitted, the said support being joined by its central part to the second arm (8) and capable of pivoting around the second axis (yy'), the said generator and the said receiver being arranged so that the X-ray source chamber and the centre of the receiver are situated on a third axis (zz') which constantly encounters the first axis (xx') at its point of intersection with the second axis (yy').

2. Imagery equipment according to claim 1, characterised in that the patient platform (6) is fitted to form part of an articulated motorised structure comprising a support cradle (1) fitted so as to be rotationally mobile according to a horizontal axis of rotation (OO') on a base (3), the said support cradle comprising a telescopic column (5) supporting the patient platform (6) which is able to translate relative to the said support cradle, the said support cradle having an elbow part which constitutes the first arm (7) pivoting around the first axis (xx') and the second arm (8) receiving the radiographic equipment (10, 11, 12) fitted to rotate relative to the said second arm (8) according to a second axis (yy') extending in a plane containing the frame of the said elbow part.

3. Imagery equipment according to any one of the aforesaid claims characterised in that the second arm (8) has a variable length.

4. Imagery equipment according to any one of claims 1 and 2 characterised in that the radiographic equipment (10, 11, 12) is fitted so as to slide along the second arm (8).

5. Imagery equipment according to any one of claims 1 and 2 characterised in that the first arm (7) and the second arm (8) consist of a sole and same part with at least two branches, one assembled to pivot around the first axis (xx') on the support (1), the other of eventually variable length supporting the radiological equipment (10, 11, 12).

6. Imagery equipment according to any one of the aforesaid claims characterised in that the support cradle (1) is equipped with slide means (2) such that the lateral guide rails form an arc of a circle which act with the guiding means (4) of the base (3) such as slides or rollers arranged between the branches of the base (3) allowing the patient platform (6) to adopt any intermediary position between the vertical position and the horizontal position.

## Patentansprüche

1. Einrichtung zur medizinischen Bilderfassung für allgemeine oder Herz-Ader-Röntgenuntersuchungen zu diagnostischen oder therapeutischen Zwecken, bestehend aus einem Patiententisch (6), der fest mit einem vorzugsweise fembedienten Stativ montiert ist, welches wiederum eine im wesentlichen aus einem Röntgenstrahlengenerator (11), einem Röntgenbildempfänger (12), gleich einem Röntgenbildschirm oder einer Röntgenfilmkassette oder einem mit einer Fernsehgruppe verbundenen Leuchtkraftverstärker, sowie Trägerelementen für Generator und Empfänger gebildete radiographische Ausrüstung aufnimmt,
dadurch gekennzeichnet, daß das Stativ einen Sattel (1) des Patiententisches (6), vorzugsweise allgemein in halbrunder Form mobil auf einen Sockel (3) montiert, aufweist und an einem Ende mit einem Ständer (5), der den besagten Patiententisch trägt, und an seinem anderen Ende mit einem ersten Arm (7), der schwenkbar um eine erste Achse (xx') parallel zur Längsachse des Patiententisches (6) sowie der des Gerüsts des Sattels (1) montiert ist, um eine Neigung mit einem Winkel von mehr oder weniger 90° in Querrichtung in Bezug auf den Patiententisch (6) zu ermöglichen, versehen ist, der besagte erste Arm (7) kraftschlüssig mit einem zweiten Arm (8) ausgestattet ist, auf den in um eine zweite Achse (yy') quer zur ersten Achse (xx') drehbarer Weise die radiographische Ausrüstung (10, 11, 12) montiert ist, so daß eine Winkelbewegung dieser letzteren von mindestens mehr oder weniger 45° in der Schädel-Rücken-Ebene ermöglicht wird, wobei der Röntgenstrahlengenerator (11) und der Röntgenbildempfänger (12) jeweils beidseitig des Patiententisches mittels einer bogenförmigen Halterung (10), auf die sie montiert sind, gehalten werden, und das mit dem zweiten Arm (8) kraftschlüssig verbundene Mittelteil der besagten Halterung gemäß der zweiten Achse (yy') schwenken kann, und wobei der besagte Generator und der besagte Empfänger so angeordnet sind, daß sich der Fokus der Röntgenstrahlenquelle und der Mittelpunkt des Empfängers auf einer dritten Achse (zz') befinden, die ständig mit der ersten Achse (xx') an deren Schnittpunkt mit der zweiten Achse (yy') zusammentrifft.

2. Bilderfassungseinrichtung gemäß Anspruch 1, dadurch gekennzeichnet, daß der Patiententisch (6) kraftschlüssig mit einer gelenkigen motorisierten Struktur versehen ist, die aus einem gemäß einer horizontalen Achse (OO') drehbar auf einen Sockel (3) montierten Sattel (1) besteht, der besagte Sattel einen teleskopischen Ständer (5) trägt, auf dem der Patiententisch translierend beweglich in Bezug auf den besagten Sattel gelagert ist, wobei dieser Sattel ein Bogenteil trägt, das den um die erste Achse (xx') schwenkenden ersten Arm (7) darstellt, und den zweiten Arm (8), der die in Bezug auf den besagten zweiten Arm drehbar um eine zweite Achse (yy'), welche sich in eine das Gerüst des besagten Bogenteils enthaltende Ebene erstreckt, montierte radiographische Ausrüstung (10, 11, 12) aufnimmt.

3. Bilderfassungseinrichtung gemäß irgendeinem der vorhergehenden Ansprüche, dadurch gekennzeichnet, daß der zweite Arm (8) in der Länge variabel ist.

4. Bilderfassungseinrichtung gemäß irgendeinem der Ansprüche 1 und 2, dadurch gekennzeichnet, daß die radiographische Ausrüstung (10, 11, 12) gleitend entlang dem zweiten Arm (8) montiert ist.

5. Bilderfassungseinrichtung gemäß irgendeinem der Ansprüche 1 und 2, dadurch gekennzeichnet, daß der erste Arm (7) und der zweite Arm (8) aus einem und demselben Stück gebildet werden, welches mindestens zwei Schenkel aufweist, wovon einer schwenkbar um die erste Achse (xx') auf den Sattel (1) montiert ist, der andere, gegebenenfalls in der Länge variabel, die radiographische Ausrüstung (10, 11, 12) trägt.

6. Bilderfassungseinrichtung gemäß irgendeinem der vorhergehenden Ansprüche, dadurch gekennzeichnet, daß der Sattel (1) mit gleitenden Elementen (2) wie seitlichen, gebogenen Führungsschienen, die mit Führungselementen (4) des Sockels (3) zusammenwirken, wie zwischen den Schenkeln des Sockels (3) angeordneten Gleitschienen oder Rollen, die ermöglichen, den Patiententisch (6) in jede beliebige Zwischenposition zwischen der vertikalen und der horizontalen Stellung zu bringen.
